# EUROPEAN PATENT APPLICATION

(11) **EP 1 952 815 A1**
(43) Date of publication of application: **06.08.2008**
(21) Application number: 06805037.6
(22) Date of filing: 23.10.2006
(51) Int. Cl.: A61K 36/16, A61P 3/00, A61K 127/00

(54) **THE USE OF GINKGO BILOBA EXTRACT IN PREPARATION OF A COMPOSITION FOR LOWERING CHOLESTEROL**

(30) Priority: 26.10.2005 CN 200510030719
(71) Applicant: SHANGHAI BIOCHIP CO., LTD., Pudong, Shanghai 201203 (CN); SHANGHAI XINGLING SCI.&TECH. PHARMACEUTICAL CO.,.., Shanghai 200127 (CN)
(72) Inventor: GU, Xiaomei, Pudong, Shanghai 201203 (CN); XIE, Delong, Shanghai 200127 (CN); ZHANG, Qinghua, Pudong, Shanghai 201203 (CN); GAO, Qi, Shanghai 200127 (CN); LIU, Gang, Pudong, Shanghai 201203 (CN); ZHANG, Guoan, Shanghai 200127 (CN); XIAO, Huasheng, Pudong, Shanghai 201203 (CN); YANG, Hongjun, Pudong, Shanghai 201203 (CN); ZHAO, Guoping, Pudong, Shanghai 201203 (CN); ZHANG, Lu, Pudong, Shanghai 201203 (CN)
(74) Representative: Bottero, Carlo
(86) International application number: PCT/CN2006/002830
(87) International publication number: WO 2007/048333

(57) **Abstract**

The use of ginkgo biloba extract in preparation of a composition for lowering cholesterol is provided. Furthermore, the use of ginkgo biloba extract in preparation of a composition for modulating the gene expressions of key enzymes associated with cholesterol metabolism in vivo is provided. Also the use of a set of changes in gene expressions at the level of genes and proteins.

SEQUENCE LISTING

<110>  SHANGHAI BIOCHIP CO., LTD.
       SHANGHAI XINGLING SCI.&TECH. PHARMACEUTICAL CO., LTD.

<120>  THE USE OF GINKGO BILOBA EXTRACT IN PREPARATION OF A COMPOSITION
       FOR LOWERING CHOLESTEROL

<130>  058317 pcep

<150>  CN200510030719.0
<151>  2005-10-26

<160>  52

<170>  PatentIn version 3.3

<210>  1
<211>  22
<212>  DNA
<213>  artificial

<220>
<223>  primer

<400>  1
tcctgcacca ccaactgctt ag                                              22


<210>  2
<211>  22
<212>  DNA
<213>  artificial

<220>
<223>  primer

<400>  2
agtggcagtg atggcatgga ct                                              22


<210>  3
<211>  19
<212>  DNA
<213>  artificial

<220>
<223>  primer

<400>  3
gctctggagg gagtgccat                                                  19


<210>  4
<211>  20
<212>  DNA
<213>  artificial

<220>
<223>  primer

<400>  4
ggatgcactg gaaagcctca                                                 20


<210>  5
<211>  20
<212>  DNA
<213>  artificial

<220>
<223>  primer

<400>  5
acatcagctg taccatgccg                                                    20


<210>  6
<211>  21
<212>  DNA
<213>  artificial

<220>
<223>  primer

<400>  6
gccccttgaa cacctagcat c                                                  21


<210>  7
<211>  20
<212>  DNA
<213>  artificial

<220>
<223>  primer

<400>  7
aaactggtgt gaggcaacgg                                                    20


<210>  8
<211>  19
<212>  DNA
<213>  artificial

<220>
<223>  primer

<400>  8
ggcaagcgca ggtgaactt                                                     19


<210>  9
<211>  20
<212>  DNA
<213>  artificial

<220>
<223>  primer

<400>  9
tccgcatgaa tgggacagat                                                    20


<210>  10
<211>  20
<212>  DNA
<213>  artificial

<220>
<223>  primer

<400>  10
taggagtgcc atgcgggtag                                                    20


<210>  11
<211>  21
<212>  DNA
<213>  artificial

<220>
<223>  primer

<400>  11
gcatccacca tcgagatcat g                                                21

<210>  12
<211>  21
<212>  DNA
<213>  artificial

<220>
<223>  primer

<400>  12
atgaactcca cctgcaagcc t                                                21

<210>  13
<211>  21
<212>  DNA
<213>  artificial

<220>
<223>  primer

<400>  13
tgcaggacca gctccaagta g                                                21

<210>  14
<211>  20
<212>  DNA
<213>  artificial

<220>
<223>  primer

<400>  14
tgggaacatc agtcggtcgt                                                  20

<210>  15
<211>  20
<212>  DNA
<213>  artificial

<220>
<223>  primer

<400>  15
cagtcgaggc catgttcctt                                                  20

<210>  16
<211>  21
<212>  DNA
<213>  artificial

<220>
<223>  primer

<400>  16
agatgccgct ctttcgaatt c                                                21

<210>  17
<211>  20
<212>  DNA
<213>  artificial

<220>
<223>  primer

<400>  17
cacaatgcaa tccgttttgg                                                   20


<210>  18
<211>  20
<212>  DNA
<213>  artificial

<220>
<223>  primer

<400>  18
ttgaggtctg cagtttccga                                                   20


<210>  19
<211>  21
<212>  DNA
<213>  artificial

<220>
<223>  primer

<400>  19
cccatgagtt cttgcgcagt a                                                 21


<210>  20
<211>  19
<212>  DNA
<213>  artificial

<220>
<223>  primer

<400>  20
cagaatgatg gcggcaatg                                                    19


<210>  21
<211>  21
<212>  DNA
<213>  artificial

<220>
<223>  primer

<400>  21
cgaagaacca tccgattgaa g                                                 21


<210>  22
<211>  21
<212>  DNA
<213>  artificial

<220>
<223>  primer

<400>  22
ccaaacctga tggcattgtg a                                                 21


<210>  23
<211>  20
<212>  DNA
<213>  artificial

<220>
<223>  primer

<400>  23
gctgctgatt gcctccttct                                                    20


<210>  24
<211>  20
<212>  DNA
<213>  artificial

<220>
<223>  primer

<400>  24
cagcactgtg agcgctgttc                                                    20


<210>  25
<211>  21
<212>  DNA
<213>  artificial

<220>
<223>  primer

<400>  25
aatgagctcc tcattgcgtc c                                                  21


<210>  26
<211>  21
<212>  DNA
<213>  artificial

<220>
<223>  primer

<400>  26
ctgcagaatg ggctgagttt c                                                  21


<210>  27
<211>  21
<212>  DNA
<213>  artificial

<220>
<223>  primer

<400>  27
aaggtttatg ccaccctcga g                                                  21


<210>  28
<211>  19
<212>  DNA
<213>  artificial

<220>
<223>  primer

<400>  28
ttcaatccaa tggagcgca                                                     19


<210>  29
<211>  21
<212>  DNA
<213>  artificial

<220>
<223>  primer

<400>  29
acaccgcgaa gaagctgagt a                                                21

<210>  30
<211>  20
<212>  DNA
<213>  artificial

<220>
<223>  primer

<400>  30
tgggcgtaat ctcatttgca                                                  20

<210>  31
<211>  21
<212>  DNA
<213>  artificial

<220>
<223>  primer

<400>  31
aatgagctcc tcattgcgtc c                                                21

<210>  32
<211>  19
<212>  DNA
<213>  artificial

<220>
<223>  primer

<400>  32
tggccagctt tctgagcat                                                   19

<210>  33
<211>  21
<212>  DNA
<213>  artificial

<220>
<223>  primer

<400>  33
aaggtttatg ccaccctcga g                                                21

<210>  34
<211>  21
<212>  DNA
<213>  artificial

<220>
<223>  primer

<400>  34
taacgattcc cctgggttga g                                                21

<210>  35
<211>  23
<212>  DNA
<213>  artificial

<220>
<223>  primer

<400>  35
gactgctctg aacgtctgaa atg                                              23


<210>  36
<211>  22
<212>  DNA
<213>  artificial

<220>
<223>  primer

<400>  36
tttccaaata accacatgtc cc                                               22


<210>  37
<211>  21
<212>  DNA
<213>  artificial

<220>
<223>  primer

<400>  37
gtgaccccac tagacaccaa c                                                21


<210>  38
<211>  19
<212>  DNA
<213>  artificial

<220>
<223>  primer

<400>  38
ggccaaaata gaggaagcc                                                   19


<210>  39
<211>  20
<212>  DNA
<213>  artificial

<220>
<223>  primer

<400>  39
taggaggcta caacgcccat                                                  20


<210>  40
<211>  20
<212>  DNA
<213>  artificial

<220>
<223>  primer

<400>  40
ccacccaccg ttcctatctc                                                  20


<210>  41
<211>  18
<212>  DNA
<213>  artificial

<220>
<223>  primer

<400>  41
aatggcaacc agcacctc                                                    18


<210>  42
<211>  21
<212>  DNA
<213>  artificial

<220>
<223>  primer

<400>  42
catgcacaga cacattctct c                                                21


<210>  43
<211>  19
<212>  DNA
<213>  artificial

<220>
<223>  primer

<400>  43
tctccaccgt gacacaatc                                                   19


<210>  44
<211>  19
<212>  DNA
<213>  artificial

<220>
<223>  primer

<400>  44
tgtctagcaa ggcttgcat                                                   19


<210>  45
<211>  20
<212>  DNA
<213>  artificial

<220>
<223>  primer

<400>  45
agcctaagga aaccgttctg                                                  20


<210>  46
<211>  22
<212>  DNA
<213>  artificial

<220>
<223>  primer

<400>  46
catgtacaat accctcctgc at                                               22


<210>  47
<211>  21
<212>  DNA
<213>  artificial

<220>
<223>  primer

<400>  47
ttacccttct caagtatggc g                                                21

<210>  48
<211>  20
<212>  DNA
<213>  artificial

<220>
<223>  primer

<400>  48
cagggcgttg aacttgacag                                                  20

<210>  49
<211>  19
<212>  DNA
<213>  artificial

<220>
<223>  primer

<400>  49
catgaaatct gtcgcaggc                                                   19

<210>  50
<211>  19
<212>  DNA
<213>  artificial

<220>
<223>  primer

<400>  50
tttggcagat gacagggac                                                   19

<210>  51
<211>  22
<212>  DNA
<213>  artificial

<220>
<223>  primer

<400>  51
ttgttacagg aagtcccttg cc                                               22

<210>  52
<211>  22
<212>  DNA
<213>  artificial

<220>
<223>  primer

<400>  52
atgctatcac ctcccctgtg tg                                               22

## Description

### Field of the Invention

The present invention relates to the use of Ginkgo *biloba* Extract (also referred to as "Ginkgo *biloba* composition") for preparing cholesterol-reducing drugs. More particularly, the present invention relates to the changes induced by Ginkgo *biloba* Extract (GBE) in gene expression of critical enzymes involved in the synthesis of bile in liver, the GBE according to the present invention can up- or down-regulate the expression of the critical enzyme genes involved in the *in vivo* cholesterol metabolism, specifically, cholesterol 7α-hydroxylase gene (Cyp7a1), 3-hydroxy-3-methylglutaryl-Coenzyme A reductase gene (Hmgcr), peroxisome proliferator-activated receptor genes (Ppars) and retinoid X receptor genes (Rxrs).

### Background Art

Ginkgo *biloba* L. belongs to Ginkgoaceae, Ginkgo *biloba.* Ginkgo leaves have long been used as herbs for its ability of recruiting lung functions, providing antitussive effect and clarifying the exudates, and therefore used for treating disorders such as asthma induced by compromised lung function, coronary heart disease and angina. It has been shown that the main active ingredients in Ginkgo *biloba* leaves include flavonoids and lactone compounds. Ginkgo *biloba* Extract enriched in the active ingredients have been prepared, for example, as disclosed in German Patents DE-B 1767 098, DE-B 2117 429, US Patent No. 5,399,348 and Chinese Patent Publication CN 1145230.

Ginkgo *biloba* Extract, GBE, has become a globally favorite as a tonic food supplement or a pharmaceutical preparation. In the middle 1960's, German scientists found that GBE can advantageously prevent or treat the cardio- and cerebro-vascular diseases and peripheral circulation disturbance. Through years of studies, GBE has been found to have the following activities: (1) dilating the vascular and increasing the blood supply; (2) decreasing the blood viscosity and improving blood hemorheology; (3) providing Anti-PAF action and inhibiting platelet agglutination and thrombosis; (4) enhancing mitochondrial functions and promoting the energy supply for neurons; (5) inhibiting oxygen radicals and decreasing the injuries induced by ischemic re-perfusion; (6) modulating the neuromediators and their receptors, and protecting neurons; (7)combating neuroimmuno-inflamation and hydrops, and protecting myelin sheath; etc.. Though there have been a number of reports on the effects of GBE in hydrocarbon metabolism, most of them are limited to the level of organism or cells. Changes in the target cells, especially the gene expression profiles in the cells, and the possibly affected metabolic paths are not elucidated on molecular level.

Further, though Ginkgo *biloba* Extract has been suggested as playing a role in lipid (e.g., triglycerides) metabolism, its action and effects in cholesterol reduction has not been reported.

Cholesterol is now pin pointed as a major cause of cardiovascular diseases. Increased total blood cholesterols is blamed as an independent risk factor of coronary heart disease, and it is also positively correlated with coronary artery pathology.

There has been a persisting need for a composition, preferably, derived from natural source, that has defined action and definite efficacy in reducing the cholesterol level, so as to effectively prevent and treat diseases associated with a high cholesterol level.

### Contents of the Invention

One object of the present invention is to provide a composition from natural source, which can effectively modulate expression of the genes involved in cholesterol metabolism and reduce the cholesterol level.

The other object of the present invention is to provide a combination useful as an action target of a cholesterol-controlling drug, which can be used as markers in drug screening.

In a first aspect, the present invention provides use of a flavanoids and lactones -enriched Ginkgo *biloba* Extract for preparing a cholesterol-reducing composition.

In a preferred embodiment, said flavanoids and lactones-enriched Ginkgo *biloba* Extract comprises 5-50% flavonoids from Ginkgo leaves, 1-10% lactones from Ginkgo leaves.

In another preferred embodiment, said flavanoids and lactones-enriched Ginkgo *biloba* Extract shows the following characteristic peaks in its fingerprint profile: flavon glycoside, rutin, shikimic acid, canine ornithine, catechins, and flavanoids.

In a further preferred embodiment, said extract is GBE50, GBE78, or the combination thereof.

In a further preferred embodiment, said composition is in the form of tablet, capsule, soft capsule, granules, drop pills, oral solution, injectable powder, lyophilized powder and injection.

In a further preferred embodiment, said composition further comprises an agent selected from the group consisting of statins and inhibins.

In a further preferred embodiment, said statin or inhibin agent is selected from the group consisting of: atorvastatin, simvastatin, lovastatin, pravastatin, fluvastatin and lipitor.

In a further preferred embodiment, said composition is further used for (a) decreasing the expression of cholesterol 7α-hydroxylase gene (Cyp7a1); (b) decreasing the expression of 3-hydroxy-3-methylglutaryl-Coenzyme A reductase gene (Hmgcr); (c) increasing the expression of peroxisome proliferator-activated receptor genes (Ppars); or (d) increasing the expression of retinoid X receptor genes.

In a further preferred embodiment, said composition is a pharmaceutical composition or a tonic (health product) composition.

In a second aspect, the present invention provides the use of flavanoids and lactones-enriched Ginkgo *biloba* Extract for preparing a composition for:
(a) decreasing the expression of cholesterol 7α-hydroxylase gene (Cyp7a1);
(b) decreasing the expression of 3-hydroxy-3-methylglutaryl-Coenzyme A reductase gene (Hmgcr);
(c) increasing the expression of peroxisome proliferator-activated receptor genes (Ppars);
(d) increasing the gene expression of retinoid x receptor (Rxr) genes; or
(e) a combination of two or more of the above.

In a third aspect, the present invention provides the use of a changed profile of gene expression at gene and protein level as an indicator of modulation on cholesterol level, wherein the change of gene expression is selected from the group consisting of:
(a) the expression of cholesterol 7α-hydroxylase gene (Cyp7a1);
(b) the expression of 3-hydroxy-3-methylglutaryl-Coenzyme A reductase gene (Hmgcr);
(c) the expression of retinoid X receptor genes (Rxrs);
(d) the expression of peroxisome proliferator-activated receptor genes (Ppars); or
(e) a combination of two or more of the above.

In a further preferred embodiment, said changed profile includes a change selected from the group consisting of:
(a) decreased expression of cholesterol 7α-hydroxylase gene (Cyp7a1);
(b) decreased expression of 3-hydroxy-3-methylglutaryl-Coenzyme A reductase gene (Hmgcr);
(c) increased expression of retinoid X receptor genes; and/or
(d) increased expression of peroxisome proliferator-activated receptor genes (Ppars).

Experimental data disclosed in the present invention shows that the gene expression changes and their combinations mentioned above have significant impacts on the cholesterol level *in vivo.*

In a further preferred embodiment, these changes and their combinations are used as indicators of the modulation on cholesterol level upon the administration of a drug, formulation, chemical agent and/or health product.

### Modes for Carrying out the Invention

Through intensive studies, the present inventor found that flavanoids and lactones-enriched Ginkgo *biloba* Extract has a significant cholesterol-reducing activity, and Ginkgo *biloba* Extract influences the gene expression of critical enzymes involved in the synthesis of bile in liver, as determined by gene chip. Specifically, flavanoids and lactones-enriched Ginkgo *biloba* Extract can inhibit the expression of the cholesterol 7α-hydroxylase gene (Cyp7a1) and the 3-hydroxy-3-methylglutaryl-Coenzyme A reductase gene (Hmgcr), but promote the expression of the peroxisome proliferator-activated receptor genes (Ppars) and retinoid X receptor genes (Rxrs), all of which genes are involved in cholesterol metabolism *in vivo.* Therefore, flavanoids and lactones-enriched Ginkgo *biloba* Extract is particularly useful in the development of cholesterol-reducing drugs and health products. These set the basis of the present invention.

### Active Ingredients

As used in the present invention, the active ingredients refer to flavanoids and lactones-enriched Ginkgo *biloba* Extract or Ginkgo *biloba* composition.

As used herein, the terms of "flavanoids and lactones-enriched Ginkgo *biloba* Extract", "Ginkgo *biloba* composition", "Ginkgo *biloba* Extract"and"flavonoids-enriched Ginkgo *biloba* extract" are used interchangeably as referring to an extract from the leaves of Ginkgo *biloba* comprising flavonoids in an amount of above 20wt%, preferably above 30wt%, more preferably above 50wt%, while not more than 80wt%. In the present invention, the flavanoids and lactones-enriched Ginkgo *biloba* extract may optionally comprise, in addition to flavonoids, lactones. Typically, the lactones are present in the flavanoids and lactones-enriched Ginkgo *biloba* Extract in an amount of about 4-10wt%.

Additionally, the amount of ginkgo acid is suitablly less than 20ppm, preferably less than 10ppm, and more preferably less than 5ppm in said flavanoids and lactones -enriched Ginkgo *biloba* Extract,.

An example of a preferred flavanoids and lactones -enriched Ginkgo *biloba* Extract is the Ginkgo *biloba* composition disclosed in Chinese Patent ZL99803683.8.

A particularly preferred flavanoids and lactones -enriched Ginkgo *biloba* Extract according to the present invention comprises 44%-78% of flavonoid compounds, 2.5%-10% of Ginkgo *biloba* lactones including Ginkgolides A, B, C, J or any of their combinations, 2.5%-10% of bilobalide and ginkgo acid at the level of 0.1-5ppm.

Preferably, said flavonoid compounds include flavonol, flavanol and flavone glycoside. And more preferably, flavone glycoside is present in an amount of 20-75%.

A particularly preferred flavanoids and lactones -enriched Ginkgo *biloba* Extract comprises 44%-78% of flavonoid compounds (including flavonol, flavanol and flavone glycoside), 2.5%-10% of Ginkgo *biloba* lactones (including Ginkgolides A, B, C, J or any of their combinations), 2.5%-10% of Bilobalide and ginkgo acid at the level of 0.1-5ppm.

A more preferred flavanoids and lactones -enriched Ginkgo *biloba* Extract comprises 44%-78% of flavonoid compounds (including 20-75% of flavone glycoside), 2.5%-10% of Ginkgo *biloba* lactones (including Ginkgolides A, B, C, J or any of their combinations), 2.5%-10% of Bilobalide and ginkgo acid at the level of 0.1-5ppm.

More preferably, the preferred Ginkgo *biloba* composition shows 17 characteristic peaks in its fingerprint, wherein the peaks include but are not limited to those of flavon glycoside, rutin, shikimic acid, anthocyanidins, catechins, flavanoids, etc..

The flavanoids and lactones -enriched Ginkgo *biloba* Extract may be prepared as previously taught in, for example, German Patents DE-B 1767 098 and DE-B 2117 429, US Patent No. 5,399,348 and Chinese Patent Publication CN1145230.

### Composition and Administration

In a further aspect, the present invention provides a composition comprising the flavanoids and lactones -enriched Ginkgo *biloba* Extract.

In the present invention, the pharmaceutical composition or the tonic composition according to the present invention may be prepared by any suitable methods as known in the art which typically include the step of mixing the flavanoids and lactones -enriched Ginkgo *biloba* Extract with a pharmaceutically acceptable carrier, excipient, diluent or any of their combinations. These agents include but are not limited to saline, buffer, glucose, water, glycerin, ethanol, or any of their combinations.

The pharmaceutical composition or the tonic composition according to the present invention may be a solid, such as granules, tablet, lyophilized powder, suppository, capsule and sublingual tablet; a liquid, such as an oral solution; or any other suitable forms. In the present invention, the active nucleic acid ingredients are typically present in an amount of about 1-99%, preferably 2-95%, more preferably about 5-90% and most preferably about 10-80%, based on the total weight of the composition.

The pharmaceutical composition or the tonic composition according to the present invention may be provided as a unit dosage or multiple dosages for administration at about 1-100mg per dose, preferably about 2-500mg per dose and even more preferably about 5-100mg per dose.

The pharmaceutical composition of the present invention may be administrated in any conventional manners as suitable, which include but not are limited to oral administration, intramuscular injection and subcutaneous injection. Preferred is oral administration. Suitable formulations may be prepared according to the selected manner of administration. Typically, the administration amount, based on the active ingredients, is about 0.01-500mg/kg body weight/day, and preferably about 0.1-50mg/kg body weight/day. The cholesterol-reducing formulation of the present invention may also be used in combination with other therapeutic agents such as statins, e.g., atorvastatin, simvastatin, lovastatin, pravastatin and fluvastatin) and/or inhibins, e.g., Lipitor; or any of their combinations.

### The advantages of the present invention include:

(a) providing a composition from natural source, which is effective to decrease cholesterol level; clarifying the mechanism that GBE decreases cholesterol level by regulating expression of four genes involved in cholesterol mechanism; determining the definite efficacy and the compatibility; and
(b) providing inhibitory action on the expression of cholesterol 7α-hydroxylase gene (Cyp7a1) and 3-hydroxy-3-methylglutaryl-Coenzyme A reductase gene (Hmgcr) and the enhancement on the expression of peroxisome proliferator-activated receptor genes (Ppars) and retinoid X receptor genes (Rxrs), which, alone or in combination, may be indicative of the metabolic level of cholesterol *in vivo,* wherein the changes in the expression profile of these genes may be utilized in, for example, identifying and screening drugs or food supplement for desired effects.

More features and benefits of the present invention will become obvious through the following illustrative and non-limiting examples. All the experiments and operations were carried out, unless otherwise specified, under the standard conditions as previously taught in, for example, the "Molecular Cloning: A Laboratory Manual" by Sambrook et al., (New York: Cold Spring Harbor Laboratory Press, 1989), or by following the manufacturer's suggestion.

### Examples

### General Methods and Materials

### 1) Construction of the animal models

(1) Sources of Animals
Wistar rats, even numbers in both genders, 3 weeks old, clean grade, purchased from the Experiment Animal Center of the Shanghai Life-Science Institute, the China Academy of Science.
(2) Diet
Basic feed was the pellet formulation for experiment rats purchased from the Experiment Animal Center of the Shanghai Life Science Institute, the China Academy of Science, the high-fat formulation and high-fat+GBE50 formulation were formulated by the feed plant of the Experiment Animal Center of the Shanghai Life Science Institute, the China Academy of Science.

**Table 1 Feed Formulations**

| **Feed** | **Formulation** |
|---|---|
| Basic | 22.08% of protein, 5.3% of fat, 4.24% of cellulose, 5.98% of ash, 1.21% of Ca, 0.75% of P, 8.8% of water |
| High-Fat | 84.5% of Basic formulation, 0.5% of sodium cholate, 10% of yolk powder, 5% of lard |
| High-Fat +GBE50 | 99.95% of High-Fat formulation, GBE 0.05% |

(3) Grouping and treatment
After one week of adaption, the rats were grouped as indicated in Table 2. The rats were maintained at a temperature of about 22±2°C, a humidity of about 60%~ 80%, allowed free to food and water, under natural illumination with a dark/light circle of about 12h.

**Table 2 Grouping and Treatment**

| # | **Groups** | **Animals** | **Treatment** |
|---|---|---|---|
| I | Normal Control | ♀x10, ♂x10 | 17 weeks on basic formulation |
| II | High-Fat Model | ♀x10, ♂x10 | 17 weeks on high-fat formulation |
| III | High-Fat +Vitamin D | ♀x10, ♂x10 | 13 weeks on high-fat formulation, followed byan intraperitoneal injection of 600 thousand Units of Vitamin D in lump |
| IV | High-Fat+GBE50 | ♀x10, ♂x10 | 17 weeks on high-fat formulation |
| V | High-Fat +Vitamin D +GBE50 | ♀x10, ♂x10 | 13 weeks on high-fat formulation, followed by a intraperitoneal injection of 6 million Units of Vitamin D in lump, and, ever since then, gastric perfusion of the Extract (100mg kg⁻¹d⁻¹) on a daily basis for 4 weeks |

(4) Administration
Preventive administration: The efficacy of GBE Via preventive oral administration was examined in rats fed on a homogeneous mixture of High-fat formulation and GBE for 30 weeks, with the groups of normal diet, High-fat diet and normal diet + GBE as the controls.
Therapeutic administration: The rats were fed on High-fat diet for 26 weeks, and then given an intraperitoneal injection of 600 thousand Units of Vitamin D in lump to induce atherosclerotic focuses *in vivo* (see, WEN, Jinquen et al., Journal of China Gerontology, 2001(21), pages 50-52; Julia B.,J. et al, Atherosclerosis, 1996(122), ps.141-152). Even since then, the extract was administrated via gastric perfusion for 4 weeks at a dosage of 50mgkg⁻¹d⁻¹. The controls were the groups on normal diet or High-fat diet plus Vitamin D, but non-treatment. The therapeutic effects of GBE were examined.
(5) Pathology Examination and Analysis
As shown in the conventional HE staining, focuses of fatty liver were observed in the animals of high-fat group and high-fat +Vitamin D group, suggesting that both high-fat diet and high-fat diet plus a lump injection of Vitamin D can induce fatty liver focuses in rats. In the group receiving Vitamin D, wall proliferation of cardio-coronary vessel, wall thickening and calcareous deposit in thoracic aorta and common carotid artery stenosis were also observed, suggesting that Vitamin D prompted the occurrence and development of the focuses in the organs such as thoracic aorta, common carotid artery and heart to an intermediate stage of atherosclerosis as pathologically diagnosed.

### 2) Cell Culture and Treatment

(1) Cell Source: HepG2 ATCC
(2) Condition of Culture
   Medium: MEM (Minimum essential medium)+10%FBS
   Incubation: 37°C, 5%CO₂
   Passage and Collection: 0.05% trypsin+0.02%EDTA
(3) Cell Treatment:
   Cells were cultured in petri dishes of Φx10cm (falcon) and grown to the desired proliferation, then detached and transferred to a 6-well plate (falcon). The cells were allowed to adhere and proliferate to the desired density (about 60%-70% confluence), and then treated and grouped as indicated in the table below.

In order to minimize the interference from the cytokines and lipids in serum without sacrificing the growth, the medium was replaced with MEM+2%FBS during treatment.

For every batch of cells, each treatment was repeated in 6 replicates. The cells were treated at different time points and collected at the same time.

**Table 3 Treatment and Grouping of Cells**

| # | **Group** | **Treatment** |
|---|---|---|
| Blank | Blank Control | 48-hr culture in normal medium |
| DMSO | Negative Control | 48-hr culture in normal medium supplemented with 0.05%DMSO |
| 2h | GBE50 treatment for 2 hrs | 2-hr culture in normal medium supplemented with 100µg/ml (final con.) GBE50 in DMSO |
| 6h | GBE50 treatment for 6 hrs | 6-hr culture in normal medium supplemented with 100µg/ml (final con.) GBE50 in DMSO |
| 12h | GBE50 treatment for 12 hrs | 12-hr culture in normal medium supplemented with 100µg/ml (final con.) GBE50 in DMSO |
| 24h | GBE50 treatment for 24 hrs | 24-hr culture in normal medium supplemented with 100µg/ml (final con.) GBE50 in DMSO |
| 48h | GBE50 treatment for 48 hrs | 48-hr culture in normal medium supplemented with 100µg/ml (final con.) GBE50 in DMSO |
| Lova | Positive Control | 6-hr culture in normal medium supplemented with 1µM lovastatin (final con.) |

### Example 1 Preparation of GBE50

GBE50 (batch No. 20030608) was purchased from Shanghai Xinglin Technology and Pharmaceutical Ltd.. The extract was prepared as previously taught in Chinese Patent ZL99803683.8, and quality controlled by adhering the National Pharmaceutical Standard WS3-227(Z-028)-2002(Z).

As tested, GBE50 comprised Ginkgo flavone glycoside ≥ 24%, whole Ginkgo flavonoid≥44%, whole Ginkgo lactones≥6%, definite active ingredients 50%, ginkgo acid ≤5PPM.

### Example 2 The effects of GBE50 on the cholesterol level in blood and in HepG2 cells

The cholesterol test kit from Shanghai JieMen Biotechnology Joint-Corporate was used in accordance to the manufacturer's instruction. Statistical Results of cholesterol levels in rats and HepG2 cells were summarized in Table 4 and Table 5.

**Table 4 Statistical Results of Cholesterol Levels in Rats of Different Treatment Groups**

| **Groups** | **Measurement: Blood Cholesterol (mol/L)** |
|---|---|
| Normal Control | 1.64±0.06 |
| High-Fat | 2.13±0.02* |
| High-Fat+GBE50 | 1.86±0.11 |
| High-Fat +V_{D} | 2.52±0.13* |
| High-Fat +V_{D}+GBE50 | 1.92±0.03 |

| | |
|---|---|
| *P<0.01 | |

**Table 5 Statistical Results of Cholesterol Levels in HepG2 Cells after different time of treatment**

| **Group** | **Measurement: Cellular cholesterol (µg/mg protein)** |
|---|---|
| Negative Control | 40.30±1.72 |
| 6-hr treatment with GBE50 | 38.22±2.36* |
| 12-hr treatment with GBE50 | 36.62±1.50* |
| 24-hr treatment with GBE50 | 29.91±1.14* |
| 48-hr treatment with GBE50 | 30.96±2.83* |
| Positive Control | 34.23±2.38* |

| | |
|---|---|
| *P<0.01 | |

Statistical results of blood cholesterol in rats showed: the cholesterol levels in the high-fat group and the high-fat +Vitamin D group were significantly higher than in the normal group and the medicated group. In the high-fat+GBE50 group and the high-fat +Vitamin D+GBE50 group, the blood cholesterol significantly decreased from the initial level before medication (P<0.01). These results suggest that GBE50 can effectively decrease the blood cholesterol in rats.

Similar results were also observed with GBE78.

Cholesterol levels in HepG2 cells also showed that treatment with GBE50 significantly reduced the cellular cholesterol (P<0.01), to some extent, in an time-dependent manner.

### Example 3 Detection of GBE's Influence on the Gene Expression Profile in liver in Rat Model

### Microarray Detection

1) Quality Control of RNA
(1) In the agarose electrophoresis image, a light contrast of 28S to 18S at 2:1 preliminarily suggested high quality of total RNA.
(2) The quality of total RNA was further examined in Agilent 2100 analyzer. A ratio of peak area of 28S to 18S at or above 2 suggested that the RNA was qualified for the next experiments.

2) Preparation and Purification of cDNA Probes
This was done by following attached protocols of SBC Rat cDNA V1.3 Array-Chip from Shanghai BioChip Company.
3) Microarray Hybridization
(1) Scheme of Microarray Detection: Liver RNA from male rats were collected, and microarray detection was performed as indicated in Table 6.
(2) Detection by SBC Rat cDNA V1.3 Chip, hybridization and scanning were run in accordance to the manufacturer's instruction.

**Table 6 Scheme of Microarray Detection**

| **No.** | **Purpose** | RNA **Source** | **Cy3** | **Cy5** | **Number of Microarrays** |
|---|---|---|---|---|---|
| 1 | Individually differential gene screening | I♂ rat liver of normal control I♂-LMIX a pool of aliquots of rat liver of normal control | I♂-1L | I♂-LMIX | 3 |
| | | | I♂-21L | I♂-LMIX | 3 |
| | | | I♂-31L | I♂-LMIX | 3 |
| | | | I♂-4L | I♂-LMIX | 3 |
| | | | I♂-5L | I♂-LMIX | 3 |
| 2 | The effects of high-fat diet on the gene expression profile in rat liver | II♂ rat liver of high-fat group I♂-LMIX a pool of aliquots of rat liver of normal control | II♂-1L | I♂-LMIX | 3 |
| | | | II♂-2L | I♂-LMIX | 3 |
| | | | II♂-3L | I♂-LMIX | 3 |
| | | | II♂-4L | I♂-LMIX | 3 |
| | | | II♂-5L | I♂-LMIX | 3 |
| 3 | The effects of high-fat diet+V_{D} on the gene expression profile in rat liver | III♂ rat liver of high-fat+V_{D} group I♂-LMIX a pool of aliquots of rat liver of normal control | III♂-1L | I♂-LMIX | 3 |
| | | | III♂-2L | I♂-LMIX | 3 |
| | | | III♂-3L | I♂-LMIX | 3 |
| | | | III♂-4L | I♂-LMIX | 3 |
| | | | III♂-5L | I♂-LMIX | 3 |
| 4 | The effects of high-fat diet+GBE50 on the gene expression profile in rat liver | VI♂ rat liver of high-fat+GBE50 group I♂-LMIX a pool of aliquots of rat liver of normal control | VI♂-1L | I♂-LMIX | 3 |
| | | | VI♂-2L | I♂-LMIX | 3 |
| | | | VI♂-3L | I♂-LMIX | 3 |
| | | | VI♂-4L | I♂-LMIX | 3 |
| | | | VI♂-5L | I♂-LMIX | 3 |
| 5 | The effects of high-fat diet+V_{D}+GBE50 on the gene expression profile in rat liver | V♂: rat liver of high-fat+V+GBE50 group I♂-LMIX: a pool of aliquots of rat liver of normal control | V♂-1L | I♂-LMIX | 3 |
| | | | V♂-2L | I♂-LMIX | 3 |
| | | | V♂-3L | I♂-LMIX | 3 |
| | | | V♂-4L | I♂-LMIX | 3 |
| | | | V♂-5L | I♂-LMIX | 3 |

### Statistical Analysis

The Microarrays were scanned using Agilent scanner. The obtained composite bi-color fluorescent image was resolved into mono-color fluorescent images using the software of TiffSplit. The images were input and analyzed using the software of Imagene 5.0. Alignment was performed automatically and manually to locate the hybridization. The background noise was filtered, and the fluorescence intensity values reflecting the gene expression were recorded and output in list. Thereby, the scan images were translated into quantitative values. The output was analyzed using the software Genespring and normalized by LOWESS to give the calculated Ratios (the ratio of cy3/cy5). The data of the microarrays were analyzed using the software of SAM (Significance Analysis of Microarrays). The genes whose expression fluctuated in either direction (Ratio<0.5 or Ratio>2) among normal rat livers were filtered and determined as individually differential genes based on the analysis of the group of individually differential gene screening. Then, differential expression screening and clustering analysis were performed with the rest groups. Results showed that, the Ratios of Cyp7a1, a critical enzyme involved in the synthesis of choleric acid in liver, in the two groups treated by GBE50 were 0.254 and 0.345 respectively, suggesting that GBE50 down-regulated the gene expression. No change in expression level was observed in the groups of high-fat and high-fat +VD (See Table 7).

**Table 7 Ratios obtained in Microarrays of Rat Liver Cyp7a1 in Different Treatments**

| Gene | Accession No. | Function | Groups | | | | |
|---|---|---|---|---|---|---|---|
| | | | Individual Differences | High-Fat | High-Fat +VD | High-Fat +GBE50 | High-Fat +VD+GBE50 |
| Cyp7a1 | NM_ 012942 | limiting enzyme in synthesis of choleric acid | 1.283 | 0.916 | 1.300 | 0.254 | 0.345 |

### Example 4 Fluorescent Quantitative PCR to Verify the Effects of GBE on the Gene Expression Profile in Liver in Rat Model

Quantitative PCR was performed in each of the treatment groups to determine the mRNA level of the genes involved in synthesis, metabolism and transportation of cholesterol, using Rat Glyceraldehyde-3-Phosphate Dehydrogease gene (Gapd) as control. Comparison was made between the treatment groups and the normal. The information of the genes verified was shown in Table 8, and results of the treatment and normal groups were summarized in Table 9.

**Table 8 Information of Genes Verified by Quantitative PCR**

| **Gene** | **Accession No.** | **Forward Primer (5'-3')** | **Reverse Primer (5'-3')** |
|---|---|---|---|
| Gapd | NM_017008 | 1294TCCTGCACCACCAA CTGCTTAG | 1395AGTGGCAGTGATGG CATGGACT |
| Cyp7a1 | NM_012942 | 1015GCTCTGGAGGGAGT GCCAT | 1115GGATGCACTGGAAA GCCTCA |
| Hmgcr | NM 013134 | 2422ACATCAGCTGTACC ATGCCG | 2524GCCCCTTGAACACC TAGCATC |
| Ldlr | NM_175762 | 2145AAACTGGTGTGAGG CAACGG | 2248GGCAAGCGCAGGT GAACTT |
| Lcat | NM_017024 | 1202TCCGCATGAATGGG ACAGAT | 1303TAGGAGTGCCATGC GGGTAG |
| Nrlh2 | NM_031626 | 1117GCATCCACCATCGA GATCATG | 1250ATGAACTCCACCTG CAAGCCT |
| Nrlh3 | NM_031627 | 1133TGCAGGACCAGCTC CAAGTAG | 1233TGGGAACATCAGTC GGTCGT |
| Nrlh4 | NM_021745 | 1133CAGTCGAGGCCATG TTCCTT | 1234AGATGCCGCTCTTT CGAATTC |
| Ppara | NM_013196 | 879CACAATGCAATCCGT TTTGG | 988 TTGAGGTCTGCAGTTTC CGA |
| Ppard | NM_013141 | 1190CCCATGAGTTCTTG CGCAGTA | 1323 CAGAATGATGGCGGCA ATG |
| Pparg | NM_013124 | 538CGAAGAACCATCCG ATTGAAG | 680CCAAACCTGATGGC ATTGTGA |
| Rxra | NM_012805 | 1103GCTGCTGATTGCCT CCTTCT | 1203CAGCACTGTGAGCG CTGTTC |
| Rxrb | NM_206849 | 913AATGAGCTCCTCATT GCGTCC | 1018CTGCAGAATGGGCT GAGTTTC |
| Rxrg | NM_031765 | 1416AAGGTTTATGCCAC CCTCGAG | 1525TTCAATCCAATGGA GCGCA |
| Acat1 | NM_017075 | 607ACACCGCGAAGAAG CTGAGTA | 723TGGGCGTAATCTCAT TTGCA |
| Acat2 | NM_009338 | 913AATGAGCTCCTCATT GCGTCC | 639TGGCCAGCTTTCTGA GCAT |
| Acatn | NM_022252 | 1416AAGGTTTATGCCAC CCTCGAG | 940TAACGATTCCCCTGG GTTGAG |

**Table 9 Results of Fluorescent Quantitative PCR**

| **Gene** | **Accession No.** | **Gene Product** | **Groups** | | | |
|---|---|---|---|---|---|---|
| | | | **High-Fat** | **High-Fat +GBE50** | **High-Fat +VD** | **High-Fat +VD +GBE50** |
| Cyp7a1 | NM_012942 | limiting enzyme in synthesis of choleric acid | 1.371±0.033 | 0.323±0.001** | 0.815±0.255 | 0.314±0.021** |
| Hmgcr | NM_013134 | limiting enzyme in synthesis of cholesterol | 0.518±0.114 | 0.321±0.054** | 1.031±0.461 | 0.269±0.051** |
| Ldlr | NM_175762 | LDL Receptor | 1.116±0.111 | 0.892±0.220 | 1.761±0.072 | 0.473±0.055* |
| Lcat | NM_017024 | lecithin-cholesterol acyl transferase | 1.508±0.201 | 0.871±0.051 | 1.025±0.024 | 1.277±0.178 |
| Nr1h2 | NM_031626 | Liver X Receptor β | 2.255±0.529 | 1.545±0.060 | 1.961±0.028 | 2.073±0.405 |
| Nrlh3 | NM_031627 | Liver X Receptor a | 1.106±0.118 | 0.983±0.171 | 1.711±0.208 | 0.660±0.008 |
| Nrlh4 | NM_021745 | Farnesol X Receptor | 1.201±0.003 | 1.365±0.264 | 1.615±0.005 | 0.912±0.134 |
| Ppara | NM 013196 | peroxisome proliferator-activated receptor α | 1.124±0.006 | 0.940±0.045 | 1.925±0.062 | 1.116±0.134 |
| Ppard | NM_013141 | peroxisome proliferator-activated receptor β | 2.330±0.127 | 5.492±0.226 *** | 5.481±0.280 | 9.918±0.236 *** |
| Pparg | NM_013124 | peroxisome proliferator-activated receptor γ | 1.651±0.111 | 1.600±0.083 | 1.709±0.188 | 1.221±0.131 |
| Rxra | NM_012805 | retinoid X receptor α | 2.054±0.085* | 1.425±0.036 | 2.858±0.296* | 1.850±0.097 |
| Rxrb | NM_206849 | retinoid X receptor β | 7.188±.053 | 2.457±.121** | 8.946±0.095 | 13.61±0.511 *** |
| Rxrg | NM_031765 | retinoid X receptor γ | 1.588±0.201 | 1.173±0.040 | 1.067±0.381 | 0.801±0.069 |
| Acat1 | NM_017075 | Acetyl CoA cholesterol acyl transferase 1 | 1.642±0.112 | 1.596±0.153 | 1.472±0.112 | 1.540±0.150 |
| Acat2 | NM_009338 | Acetyl CoA -cholesterol acyl transferase 2 | 0.892±0.095 | 1.037±0.196 | 1.026±0.050 | 0.951±0.057 |
| Acatn | NM_022252 | Acetyl CoA-cholesterol acyl transporter | 1.655±0.140 | 1.020±0.022 | 1.616±0.332 | 1.428±0.188 |

| | | | | | | |
|---|---|---|---|---|---|---|
| *P<0.05, **P<0.01 and ***P<0.001 | | | | | | |

Quantitative PCR was performed to determine the mRNA levels of a series of genes in rat livers in each of the treatment groups, and statistical data were obtained as shown in Table 9. The data suggested:
(1) By comparison with the normal control, the expression of Cyp7a1 gene and Hmgcr gene was significantly decreased in High-fat diet+GBE50 group and High-Fat +VD+GBE50 group (P<0.01), while no significant difference in expression of the two genes was observed in High-Fat group and High-Fat +VD group.
(2) By comparison with the normal control, the expression of the Rxrb gene, which encode the β subtype retinoid x receptor, in each of the four treatment groups was significantly increased. Its expression in High-Fat+VD+GBE50 group was significantly higher than in High-Fat +VD group, and the expression in High-Fat group was significantly lower than in High-Fat+GBE50 group. This may be attributed to the fact that the transcription of RXR receptor can be regulated by exogenous and endogenous metabolic stimuli such as 9-*cis*-retinoic acid and certain drugs (See R. Fraydoon *et al.,* Current Opinion in Structure Biology, 2001(11), ps.33-38).
(3) The expression of liver X Receptors (LXR) α and β genes, Nrlh2 and Nrlh3, was observed in each of the groups without significant difference. The expression of Farnesol X Receptor gene Nrlh4 was observed in each of the groups without significant difference.
(4) The expression of LDL (Low Density Lipoprotein) receptor gene Ldlr is significantly lower in High-Fat+VD+GBE50 group than in High-Fat+VD group.
(5) No significant difference in the expression of lecithin-cholesterol acyl transferase gene Lcat, Acetyl CoA-cholesterol acyl transferase genes Acat1, Acat2 and Acatn among different groups was observed, suggesting that GBE50 has no effect on transcription of these genes.
(6) Extremely significant increase in the expression of Ppard, peroxisome proliferator-activated receptor genes (Ppars), was observed in High-Fat+GBE50 group and in High-Fat+VD+GBE50 group.

### Example 5 Fluorescent Quantitative PCR to Determine the Effects of GBE on the Expression Profile in the culture of human hepatic cell line HepG2

This study include selected genes involved in different stages of cholesterol metabolism and the genes exhibited significant differential expression in the liver of rat model. Fluorescent Quantitative PCR was performed to examine the changes at mRNA level in human hepatic cell line HepG2 treated by GBE50.

Human β-actin was used as control. The cells were treated as indicated in Table 3. Groups of 2-, 6-, 12-, 24- and 48-hr GBE50 treatment were compared with group of 48-hr DMSO. Information of the genes determined was shown in Table 10. Comparison was summarized in Table 11.

**Table 10 Information of the Genes Determined by Quantitative PCR**

| **Gene** | **Accession No.** | **Forward Primer (5'-3')** | **Reverse Primer (5'-3')** |
|---|---|---|---|
| ABCG5 | NM_022436 | GACTGCTCTGAACGTCTGAAATG | TTTCCAAATAACCACATGTCCC |
| ABCG8 | NM_022437 | GTGACCCCACTAGACACCAAC | GGCCAAAATAGAGGAAGCC |
| HMGCR | NM_000859 | TAGGAGGCTACAACGCCCAT | CCACCCACCGTTCCTATCTC |
| Insig2 | NM_016133 | AATGGCAACCAGCACCTC | CATGCACAGACACATTCTCTC |
| LDLR | NM_000527 | TCTCCACCGTGACACAATC | TGTCTAGCAAGGCTTGCAT |
| PPARA | NM_005036 | AGCCTAAGGAAACCGTTCTG | CATGTACAATACCCTCCTGCAT |
| PPARD | NM_006238 | TTACCCTTCTCAAGTATGGCG | CAGGGCGTTGAACTTGACAG |
| SCARB1 | NM_005505 | CATGAAATCTGTCGCAGGC | TTTGGCAGATGACAGGGAC |
| β-actin | NM_001614 | TTGTTACAGGAAGTCCCTTGCC | ATGCTATCACCTCCCCTGTGTG |

**Table 11 Results of Fluorescent Quantitative PCR**

| **Gene** | **Accession No.** | **Gene Product** | **2-hr GBE** | **6-hr GBE** | **12-hr GBE** | **24-hr GBE** | **48-hr GBE** |
|---|---|---|---|---|---|---|---|
| ABCG5 | NM_022436 | Membrane Protein, Steroid Transporter | 0.626±0.003 | 0.960±0.001 | 0.956±0.003 | 1.138±0.009 | 1.401±0.019 |
| ABCG8 | NM_022437 | Membrane Protein, Steroid Transporter | 0.470±0.003 | 1.741±0.007 | 0.835±0.007 | 0.944±0.003 | 0.832±0.006 |
| HMGCR | NM_000859 | limiting enzyme in biosynthesis of cholesterol | 0.658±0.016* | 0.506±0.037* | 0.787±0.033 | 0.890±0.032 | 1.095±0.032 |
| Insig2 | NM_016133 | Insulin-inducing protein 2 | 0.819±0.023 | 0.850±0.007 | 0.993±0.022 | 1.210±0.023 | 1.094±0.027 |
| LDLR | NM_000527 | LDL Receptor | 0.605±0.012 | 0.911±0.006 | 0.730±0.025 | 0.729±0.007 | 0.821±0.014 |
| PPARA | NM_005036 | peroxisome proliferator-activated receptor α | 1.244±0.004 | 0.775±0.006 | 0.936±0.007 | 0.857±0.002 | 1.580±0.004 |
| PPARD | NM_006238 | peroxisome proliferator-activated receptor δ | 1.133±0.002 | 0.752±0.004 | 1.044±0.003 | 1.092±0.003 | 1.754±0.005* |
| SCARB1 | NM_005505 | Scavenger Receptor B 1 | 1.017±0.155 | 0.562±0.041 | 0.857±0.043 | 1.127±0.117 | 0.818±0.039 |

| | | | | | | | |
|---|---|---|---|---|---|---|---|
| *P<0.05, **P<0.01 and ***P<0.001 | | | | | | | |

Fluorescent Quantitative PCR was performed to examine the changes in the expression of the genes involved in cholesterol metabolism in HepG2 treated by GBE50 treatment for different periods of time. The statistical results showed:

For the 2 and 6 hours of the GBE50 treatment, the expression of HMGCR, a critical enzyme in cholesterol synthesis, significantly decreases. After 48 hours of treatment, a moderate increase in the expression of peroxisome proliferator-activated receptor PPARD was observed. No significant changes were observed in the expression of LDL receptors LDLR and PPARA. The results were consistent with those observed in rat liver in animal model

The results showed that GBE50 modulates cholesterol level via HMGCR, Cyp7a1 and PPARD.

Each of the references cited in the present invention is incorporated as being individually referred to in its own entirety. It should be understood that various changes and modifications without deviating from the purposes and spirit of the invention would be obvious to anyone of ordinary skills in the art from the above description, which should all be understood as falling in the scope of the invention only defined by the claims appended hereto.

## Claims

1. Use of a Ginkgo *biloba* Extract for preparation of a cholesterol-reducing composition.

2. The use of claim 1, wherein said Ginkgo *biloba* Extract comprises 5-50% of flavonoids from Ginkgo leaves and 1-10% of lactones from Ginkgo leaves.

3. The use of claim 1, wherein said Ginkgo *biloba* Extract shows the following characteristic peaks in its fingerprint: flavon glycoside, rutin, shikimic acid, anthocyanidins, catechins and flavanoids.

4. The use of claim 1, wherein said extract is Ginkgo flavanoids and lactones, a standard Ginkgo *biloba* Extract, or a combination thereof.

5. The use of claim 1, wherein said composition is in a form selected from the group consisting of tablet, capsule, soft capsule, granules, drop pills, oral solution, injectable powder, lyophilized powder and injection.
In a further preferred embodiment, said composition further comprises an agent selected from the group consisting of statins and inhibins.
In a further preferred embodiment, said statin or inhibin agent is selected from the group consisting of: atorvastatin, simvastatin, lovastatin, pravastatin, fluvastatin and lipitor.

6. The use of claim 1, wherein said composition is further used for (a) decreasing the expression of cholesterol 7α-hydroxylase gene (Cyp7a1); (b) decreasing the expression of 3-hydroxy-3-methylglutaryl-Coenzyme A reductase gene (Hmgcr); (c) increasing the expression of peroxisome proliferator-activated receptor genes (Ppars); and/or (d) increasing the expression of retinoid X receptor genes (Rxrs).
In a further preferred embodiment, said composition is a pharmaceutical composition or a tonic composition.

7. Use of a Ginkgo *biloba* Extract for preparation of a composition for: (a) decreasing the expression of cholesterol 7α-hydroxylase gene (Cyp7a1); (b) decreasing the expression of 3-hydroxy-3-methylglutaryl-Coenzyme A reductase gene (Hmgcr); (c) increasing the expression of peroxisome proliferator-activated receptor genes (Ppars); or (d) increasing the expression of retinoid X receptor genes (Rxrs); or (e) a combination of two or more of the above.

8. Use of a changed profile of gene expression at gene and protein levels as an indicator of modulation on cholesterol level, wherein said expression is selected from the group consisting of:
(a) the expression of cholesterol 7α-hydroxylase gene (Cyp7a1);
(b) the expression of 3-hydroxy-3-methylglutaryl-Coenzyme A reductase (Hmgcr);
(c) the expression of retinoid X receptor genes (Rxrs);
(d) the expression of peroxisome proliferator-activated receptor genes (Ppars); or
(e) a combination of two or more of the above.

9. The use of claim 8, wherein said profile includes a change selected from the group consisting of:
(a) decreased expression of cholesterol 7α-hydroxylase gene (Cyp7a1);
(b) decreased expression of 3-hydroxy-3-methylglutaryl-Coenzyme A reductase gene (Hmgcr);
(c) increased expression of retinoid X receptor genes (Rxrs); and
(d) increased expression of peroxisome proliferator-activated receptor genes (Ppars);
the above changes and their combinations affect the cholesterol level *in vivo*.

10. The use of claim 8, wherein these changes or a combination thereof are used as indicators of the modulation on cholesterol level after the administration of a drug, a formulation, a chemical agent and/or a health product.
